(19) Europäisches Patentamt / European Patent Office / Office européen des brevets

(11) **EP 3 447 745 A1**

(12) **EUROPEAN PATENT APPLICATION**
published in accordance with Art. 153(4) EPC

(43) Date of publication:
**27.02.2019 Bulletin 2019/09**

(21) Application number: **17785835.4**

(22) Date of filing: **10.04.2017**

(51) Int Cl.:
*G08B 21/02* (2006.01)    *A41D 13/00* (2006.01)
*A61B 5/00* (2006.01)    *B32B 5/26* (2006.01)
*D03D 1/00* (2006.01)    *D03D 11/00* (2006.01)
*D03D 15/00* (2006.01)    *G08B 19/00* (2006.01)
*G08B 25/04* (2006.01)    *G08B 25/10* (2006.01)

(86) International application number:
**PCT/JP2017/014617**

(87) International publication number:
**WO 2017/183495 (26.10.2017 Gazette 2017/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **19.04.2016 JP 2016083585
19.04.2016 JP 2016083586
19.04.2016 JP 2016083589**

(71) Applicant: **Teijin Limited
Osaka-shi, Osaka 530-0005 (JP)**

(72) Inventors:
• **KIMURA, Tasuku**
**Osaka-shi**
**Osaka 541-0054 (JP)**
• **HAYASHI, Hirokazu**
**Osaka-shi**
**Osaka 541-0054 (JP)**

(74) Representative: **Cockerton, Bruce Roger
Carpmaels & Ransford LLP
One Southampton Row
London WC1B 5HA (GB)**

(54) **ARTICLE PROVIDED WITH WARNING SYSTEM**

(57) The present invention addresses the problem of providing a protective-equipment article provided with a warning system that can detect information on a position within a building, detect danger to human life such as hyperthermia, and issue a warning, while ensuring safety, work efficiency, and convenience. As a means for solving such problem, provided is an article equipped with a warning system, wherein the warning system includes the following: a position information sensor; a triaxial acceleration sensor, a gyro sensor, a geomagnetic sensor, or a combination thereof; and a means that can autonomously estimate a position from the information emitted from such sensors.

Fig.1

**Description**

Technical Field

The present invention is related to an article with an alarm system capable of detecting position information in a building, detecting a risk of life such as heat strokes to issue an alarm while ensuring safety, workability and convenience.

Background Art

It is important for operators who work in harsh environments to manage the physical condition of the operators during work. For example, thermal convulsions called heat stroke and fever syncope, in workplaces with dangerous work, increases risk of life of the operators. In particular, firefighters install and carry cylinders and various equipment in addition to protective equipment such as fire protection clothes, and work in high temperature environments close to the flames during firefighting activities. The heat is likely to be trapped inside the clothes, and there is a high risk that the firefighter is exposed to the risk of heat stroke. Further, the firefighter may act more than the physical strength limit due to its sense of mission. Under such an environment, it has been desired to detect and inform a state of firefighters in which the danger of heat stroke is high.

As a solution to this, for example, an earplug type one has been proposed in Patent Document 1. However, there is a problem that the hearing of the firefighter is hindered because it is an earplug type. Further, when the earplug type one is applied in a firefighting activity, durability in harsh environments is not enough.

Further, in Patent Document 2, there is proposed a method of sending information detected by a temperature sensor to an external module by a communication means and determining whether there is a danger of heat stroke in an external module. However, in this method, there is a problem that the alarm system does not work when the firefighter was in a place where communication cannot be ensured, such as inside the building or underground.

In addition, a system for detecting heat stress in firefighting activities is proposed in Patent Document 3. However, in such a method, there is a concern that the activities of firefighters may be hindered due to the characteristic of being measured at the head, and there is also a problem that the warning system does not function when the head protector is removed.

[Prior Art Document]

[Patent document]

[Patent Document 1] Japanese Laid-open Patent Publication No. 2013-048812
[Patent Document 2] Japanese Laid-open Patent Publication No. 2012-187127
[Patent Document 3] Japanese Laid-open Patent Publication No. 2004-030180

Disclosure of Invention

Problem to be Solved by Invention

The present invention has been made in consideration of the above situation, and an object of the present invention is to provide an article that is capable of detecting position information within a building, and detecting hazards related to life such as heat stroke to issue an alarm, while ensuring safety, workability, and convenience.

Means to Solve the Problem

According to the present invention, "an article including an alarm system, characterized in that the alarm system includes a position information sensor" is provided.

In this case, it is preferred that the alarm system includes, in addition to the position information sensor, a three-axes acceleration sensor, a gyro sensor, a geomagnetic sensor, or a combination thereof; and a part configured to autonomously estimate a position from information emitted from the position information sensor. Further, it is preferred that the alarm system further includes an atmospheric pressure sensor. Further, it is preferred that the alarm system further includes; a biometric information sensor that detects biometric information of a wearer of the article; a determination part configured to determine that the biological information detected by the biometric information sensor has reached a threshold value; an alarm part configured to warn that a risk is heightened in response to an instruction from the determination part; a transmission part configured to transmit a warning in response to the alarm part being operating; and a control part configured to control the alarm part and the transmission part. Further, it is preferred that the biometric information sensor is a temperature sensor that detects an inside temperature of the article.

According to the present invention, there is provided an article including an alarm system, characterized in that the alarm system includes; a reception part configured to receive the alarm transmitted from the transmission part; an alarm part; a display part; and a control part configured to control the reception part and the alarm part.

In this case, it is preferred that the alarm part is based on a voice, light, a display on a display part, a vibration of the sensor itself, or a combination thereof. Further, it is preferred that it includes a display part configured to indicate that the sensor, the determination part, the alarm part, the transmission part, or the reception part is operating normally.

In the present invention, it is preferable that the alarm system includes; a position information sensor, a calculation part configured to calculate a position uncertainty degree from information generated from the position information sensor; an alarm part configured to alarm the position uncertainty degree calculated by the calculation part; a transmission part configured to transmit the alarm upon the alarm part is activated; and a control part configured to control the alarm part and the transmission part.

In the present invention, it is preferable that the alarm system includes, in addition to the position information sensor, a three-axes acceleration sensor, a gyro sensor, a geomagnetic sensor, or a combination thereof; a part configured to autonomously estimate a position from information emitted from the position information sensor; and a part configured to increase position information accuracy within a building by using a position information detection system provided in the building.

In the present invention, it is preferred that the article is a garment constituted by a multilayer-structured cloth. Further, it is preferred that the sensor is provided on an interlayer of the multilayer-structured cloth or a surface of an innermost layer on a skin side. Further, it is preferred that the sensor is provided on an interlayer of the multilayer-structured cloth or a surface of an innermost layer on a skin side. Further, it is preferred that an outermost surface of the cloth constituting the garment has a water repellency of class 3 or higher as measured by a spray method prescribed in JIS L1092. Further, it is preferred that the cloth constituting the garment has a heat shrinkage percentage of 10% or less according to an international performance standard ISO 11613-1999. Further, it is preferred that the garment is a fire protective garment Further, it is preferred that the garment is used for one selected from a group, the group including a member of the SDF, military, rescue team, police officers, security guards, construction workers, civil engineering workers, road construction site workers, workers at blast furnaces, workers at nuclear power plants, workers at high temperatures, agricultural workers, school activists, sports athletes, amusement workers, care receivers requiring watching, infants, inpatients, doctors, nurses, caregivers, pets or livestock requiring watching. Further, it is preferred that the cloth constituting the garment includes aramid fibers.

Advantage of the Invention

According to the present invention, it is possible to provide an article that is capable of detecting position information within a building, and detecting hazards related to life such as heat stroke to issue an alarm, while ensuring safety, workability, and convenience.

Brief Description of Drawings

FIG. 1 is a diagram showing an example of an embodiment of the present invention.
FIG. 2 is a diagram showing an example of a system that can be used in the present invention.
FIG. 3 is a diagram showing an example of a flowchart usable in the present invention.
FIG. 4 is a diagram showing an example of recorded contents of an abnormality detection device 2 in the present invention.
FIG. 5 is a diagram showing an example of a display of the second alarm generating device 4 in the present invention.
FIG. 6 is a diagram schematically showing a protective garment obtained in Example 1.
FIG. 7 is a diagram showing an alarm system connected to a network.

Best Mode for Carrying Out the Invention

First, a first aspect of the present invention is an article with an alarm system, wherein the alarm system includes a position information sensor.

A second aspect is an article provided with an alarm system, wherein the alarm system includes, in addition to the position information sensor, a three-axes acceleration sensor, a gyro sensor, a geomagnetic sensor, or a combination thereof; and a part configured to autonomously estimate a position from information emitted from the position information sensor.

A third aspect is an article provided with an alarm system, wherein the alarm system includes; a position information sensor, a calculation part configured to calculate a position uncertainty degree from information generated from the position information sensor; an alarm part configured to alarm the position uncertainty degree calculated by the calculation part; a transmission part configured to transmit the alarm upon the alarm part is activated; and a control part configured to control the alarm part and the transmission part.

A fourth aspect is an article provided with an alarm system, wherein the alarm system includes, in addition to the position information sensor, a three-axes acceleration sensor, a gyro sensor, a geomagnetic sensor, or a combination thereof; a part configured to autonomously estimate a position from information emitted from the position information sensor; and a part configured to increase position information accuracy within a building by using a position information detection system provided in the building.

Hereinafter, an embodiment of the present invention will be described based on an example in which an alarm system is a heat stroke alarm system and includes means capable of autonomous estimation and applied to a protective garment (article).

Here, "autonomous estimation" is also referred to as "autonomous navigation", and it is an indoor positioning technology using a sensor as described in, for example, HCG Symposium 2014 CD-ROM paper, electronic information communication conference "pedestrian autonomous navigation (PDR) benchmark method and its evaluation". The "position information sensor" referred to in the present invention includes a GNSS sensor and the like.

It is preferable that the alarm system further includes an atmospheric pressure sensor. The atmospheric pressure sensor is preferably an atmospheric pressure sensor for detecting altitude or elevation difference.

Further, it is preferable that the heat stroke alarm system, which includes the means capable of autonomous estimation, further includes a sensor that detects the biological information of the wearer of the article, a determination part configured to determine that the risk of heat stroke has increased as the biological information detected by the sensor reaches the threshold value, an alarm part configured to warn the risk of heat stroke has increased, a transmission part configured to transmit an alarm when the alarm means is activated, and a control part configured to control the alarm part and the transmission part. Such a protective garment is suitably used, for example, as a fire-fighting protective garment (firefighting suit) for firefighters.

Here, as the sensor for detecting the biological information, it is preferable to be a temperature sensor for detecting an inside temperature of protective garment, but in addition to this, a temperature sensor for detecting an external temperature of a protective garment, a temperature sensor for detecting the internal or external humidity, a sensor for detecting oxygen concentration in blood, a sensor for detecting heart rate, a sensor for detecting an electrocardiogram, a sensor for detecting pulses, a sensor for detecting pulse waves, a sensor for detecting blood pressure, a sensor for detecting blood flow, a sensor for detecting the presence or absence of a body motion, a sensor for detecting a body position, a sensor for detecting a skin temperature, a sensor for measuring a temperature of a eardrum, a sensor for measuring a rectal temperature, a sensor for detecting a hue of skin, a sensor for detecting perspiration, a sensor for detecting the number of breaths and speed and depth of breaths, a sensor for detecting brain waves, a sensor for detecting opening of pupil, etc., can be used. Further, a combination of these two or more sensors can be used. Because heat stroke symptoms are various, it is expected to improve its detection accuracy by combining a plurality of sensors.

Hereinafter, the sensor for detecting the biological information will be described as a temperature sensor for detecting the inner temperature of the protective garment, but it goes without saying that the sensor is not limited to the temperature sensor.

In addition, the protective garment for administrator is a protective garment provided with a heat stroke alarm system, and the heat stroke alarm system includes a reception part configured to receive a warning sent from a transmission part of a worker (protective garment for firefighters for firefighters) an alarm part, and a control part configured to control the reception part and the alarm part. Such protective garment is suitably used as a protective garment (a protective garment for firefighters) for a captain of a firefighter, for example.

For example, if the firefighters and the captain of firefighters use such protective garments equipped with these heat stroke alarm systems respectively, the risk of heat stroke, including part to autonomously estimate the position, can be alarmed while ensuring safety, workability and convenience.

Hereinafter, a heat stroke alarm system, which includes a position information sensor, a three-axes acceleration sensor, a gyro sensor, a geomagnetic sensor, or a combination thereof, and a part capable of autonomously estimating a position using a temperature sensor, is described with referring to figures (see figures other than the reference drawing as appropriate.

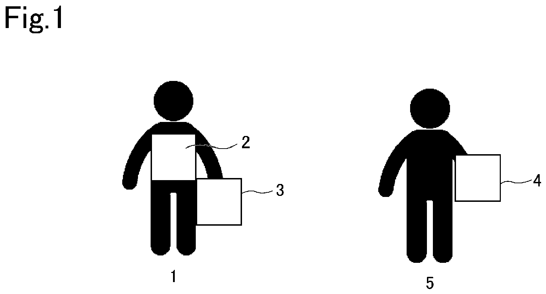

As shown in FIG. 1, a heat stroke alarm system includes an abnormality detection device 2a, 2b, 2c (2) (hereinafter, simply when not particularly distinguished referred to as "abnormality detection device 2"), which is accommodated in the inner and outer portions of each of the protective garment of the operator 1a, 1b, 1c (1) (hereinafter, simply when not particularly distinguished referred to as "worker 1") (subject), a first alarm generation device 3, and a second alarm generation device 4 held by an administrator 5 other than the worker 1.

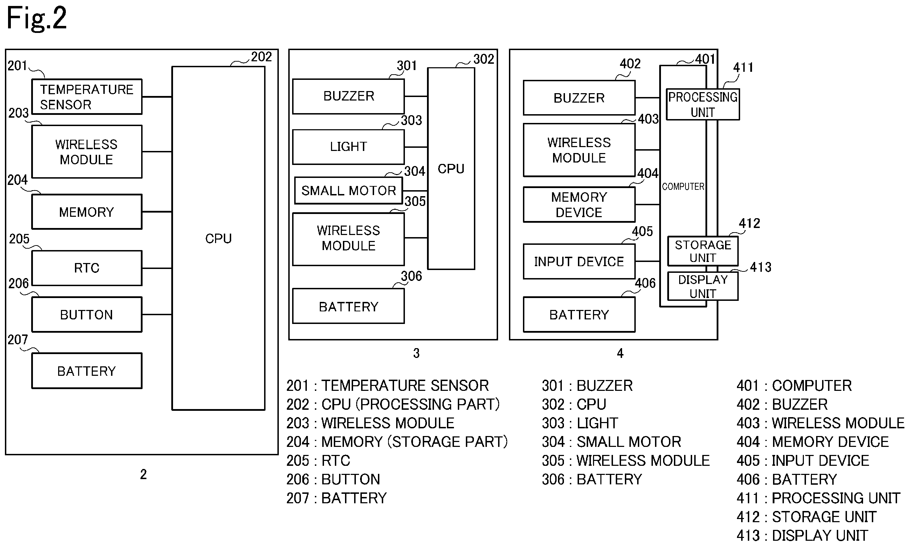

As shown in FIG. 2, the abnormality detection device 2, the sensors (a position information sensor, a three-axes acceleration sensor, a gyro sensor, a geomagnetic sensor, a pressure sensor and a temperature sensor) 201, CPU (processing unit) 202, a wireless module 203, a memory (storage part) 204, an RTC (Real Time Clock) 205, a button 206, and a battery 207. In addition, the first alarm generation device 3 may include a buzzer (abnormality notifying part) 301, a CPU (processing part) 302, a small light 303, and a small motor (abnormality notifying part) 304. Further, a CPU 303 of the alarm generation device and a CPU202 abnormality detection device 2 may be the same, for one abnormality detection unit 2, a system may be configured such that a plurality of first alarm generation device 3 may be included in the system. By including a plurality of the first alarm generation devices 3, the own worker can notice an alarm at an early stage. The sensors (the position information sensor, the three-axes acceleration sensor, the gyro sensor, the geomagnetic sensor, the pressure sensor and the temperature sensor) 201 is a part which autonomously estimates building intrusion information and the position information within the building and a part (sensor) for measuring the temperature inside clothes. Hereinafter, the data acquired by the sensor 201 will be referred to as "sensor data". The CPU 202 performs various arithmetic processing using the memory 204. The wireless module 203 is part configured to wirelessly communicate with external devices (the first alarm generation device 3 and the second alarm generation device 4). The memory 204 is storage part and can be realized by, for example, a RAM (Random Access Memory), ROM (Read Only Memory), HDD (Hard Disk Drive), or the like. The RTC 205 is a part for measuring time, and can be realized by, for example, a dedicated chip, and can operate by receiving power supply from the built-in battery even while the battery is not working. The battery 207 is a power supply part, and can be realized by, for example, a storage battery. The button 206 is an input part operated (pressed) by the worker 1. By disposing the CPU 202 as the determination part configured to determine whether the information detected by the sensor 201 and the sensor 201 is equal to or more than the threshold value, and the intrusion of buildings and the position uncertainty degree in the same unit (the abnormality detection device 2), it is possible to detect and determine the danger of heat stroke and alert the worker 1, in case where the wireless module 203 is faulty or in an environment where wireless communication cannot be used.

Next, as shown in FIG. 2, the first alarm generation device 3 includes a CPU 302, a buzzer 301, and a battery 306. The buzzer 301 is an alarm part configured to generate a buzzer sound based on an instruction from the CPU 302. The light 303 and the small motor 304 are alarm part configured to generate light and vibration in accordance with instructions from the CPU 302. In addition to the sound of the buzzer 301, an alarm by light or vibration is issued so that the worker 1 can more certainly notice the alarm more quickly. The wireless module 305 is part for wirelessly communicating with the abnormality detection device 2 and the external device (the second alarm generation device 4). The battery 306 is a power supply part, and can be realized by, for example, a storage battery.

Next, as shown in FIG. 2, the second alarm generation device 4 includes a panel type computer 401, a buzzer 402, a wireless module 403, a memory device 404, an input device 405, and a battery 406. The panel type computer 401 is a computer in which a processing unit 411 including a CPU and the like, a storage unit 412 including a RAM, a ROM, an HDD, and the like, a display unit 413 that is a liquid crystal display with a touch panel, and the like are integrally incorporated. The panel type computer 401 enables an operator to intuitively perform a manual operation on the display unit 413. The buzzer 402 is an alarm part for generating a buzzer sound based on an instruction from the panel type computer 401. The wireless module 403 is part configured to wirelessly communicate with an external device (the first alarm generation device 3). The memory device 404 is a detachable storage medium and can be realized by, for example, a flash memory. The battery 406 is a power supply part and can be realized by, for example, a storage battery.

Further, the antennas of the wireless modules 203, 305, 403 can also be integrally formed with protective garment using conductive fibers or the like. For example, if the antenna is formed on the outer surface of the protective garment, even if the abnormality detection device 2, the first alarm generation device 3, and the second alarm generation device 4 are provided inside the protective garment, wireless communication can be carried out without being hindered by the protective garment. For this reason, it is also possible to use a cloth having high electromagnetic wave absorption rate for protective garment. Antennas provided integrally with the protective garment can be formed by not only conductive fibers but also a method of depositing or printing a conductive material on protective garment by vapor deposition or printing, a method of attaching an antenna preliminarily formed in a flexible substrate such as a flexible substrate to the protective garment, for example.

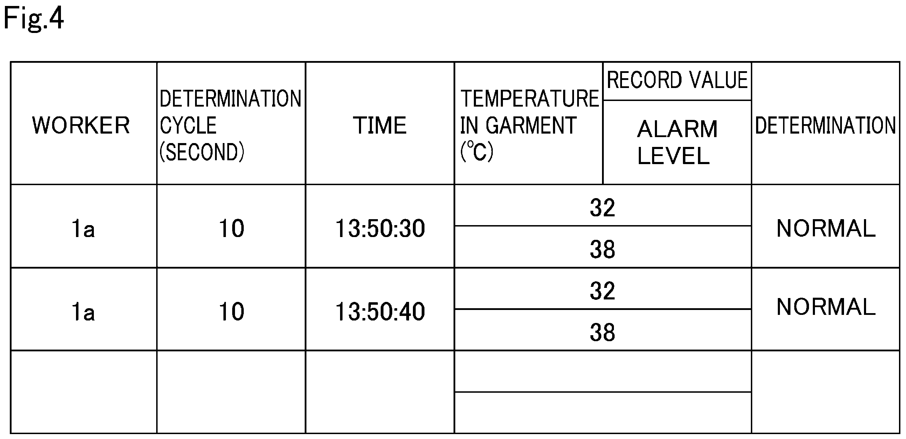

Next, an example of data stored in the storage unit 204 of the abnormality detection device 2 will be described. As shown in FIG. 4, the data in the storage part is composed of five columns and will be described in order from the left.

"Worker" indicates an identifier of the worker 1. Determination cycle (in seconds) ", a cycle (seconds) with which the abnormality detection device 2 is periodically collects data by temperature sensor 201 and stores the date in the memory 204, and a cycle (seconds) with which the data is compared with a preset threshold to determine abnormality of the body of the worker 1. For example, there are 60 seconds as a long cycle and 10 seconds as a short cycle. For example, the time at which the sensor data is collected at any time is the time measured by the RTC 205.

Regarding "Temperature in a garment (°C) ", "recorded value" in the upper row shows the temperature in the garment of the worker 1 acquired by the sensor 201 at that time as the biological information. "Warning level" of the lower part shows the temperature as a first threshold value, and is 38 °C in FIG. 4. The threshold value of various kinds of biological information may be an absolute value or a relative value. If the relative value is set as the threshold value, the risk of heat stroke can be reliably detected at an earlier stage by coping with the individual difference of the workers/0} 1 and the fluctuation of the daily physical condition. Further, with respect to a single determination items (e.g. body temperature), a relative threshold and an absolute threshold may be used in combination. In addition, the determination as to whether each item is abnormal or not may be implemented based on the amount of change in data as described above, or based on the rate of change (the amount of change per unit time) of the data.

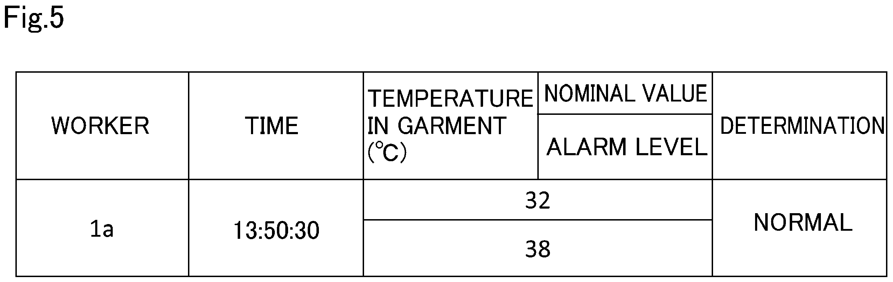

Next, an example of a screen displayed on the display unit 413 of the second alarm generation device 4 will be described. As shown in FIG. 5, on the display unit 413, as a screen for an administrator, information for identifying the worker is displayed in the leftmost column, and items such as time, temperature in a garment, and determination items are displayed in the right column.

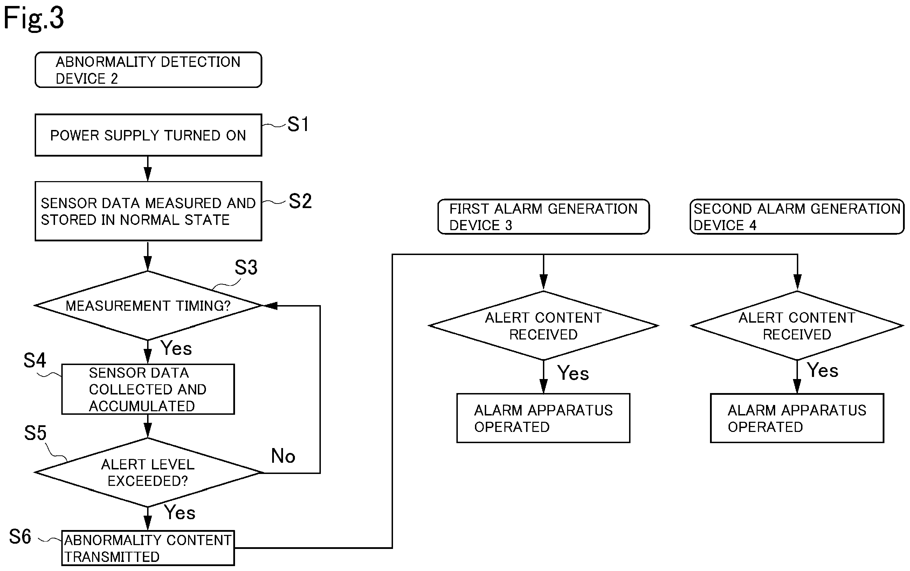

Next, the processing flow of the heat stroke alarm system will be described. Note that a plurality of the abnormality detection device 2 may be used actually; however, here it is assumed that one abnormality detection device 2 is used in order to simplify the description. As shown in FIG. 3 (appropriately with reference to other drawings), first, the abnormality detection device 2, when the power is turned on by the worker 1 (step S1), measures sensor data in the normal state before work as nominal values using the sensor 201, and stores the sensor data in the memory 204 (step S2). Thereafter, the worker 1 starts work. Next, the CPU 202 of the abnormality detection device 2, which is the determination part, determines whether or not the determination timing has arrived based on the determination cycle (step S3), and it is determined that the determination timing has arrived (Yes), it proceeds to step S4.

In step S4, the CPU 202 of the abnormality detection device 2 collects the sensor data and stores it in the memory 204. In addition, at this time, it is also possible to check the voltage of the battery 207 and the communication state with the first alarm generation device 3 or the second alarm generation device 4. Next, the CPU of the abnormality detection device 2, which is the determination part, determines whether or not the sensor data is equal to or higher than the alarm level based on the sensor data collected in the previous step S4 (whether the item "determination" is "abnormality(alarm)" (step S5), and in the case of Yes proceeds to step S6, in the case of No returns to step S3. In step S6, the CPU 202 of the abnormality detection device 2 notifies the first alarm generation device 3 of the warning content (see FIG. 3)

When receiving the warning contents from the abnormality detection device 2, the first alarm generation device 3 activates the alarm part. Specifically, for example, by sounding the buzzer 301, the worker 1 is notified of the abnormality. At the same time, the small motor 304 may be operated to generate vibration. The worker 1 can immediately recognize the occurrence of abnormality due to vibration by the small motor 304, even in a work environment where noise is large and sound of the buzzer 301 is difficult to hear. Thus, according to the heat stroke alarm system of the present embodiment, it is possible to reliably detect the danger of heat stroke of the worker 1 and the state of intrusion into the building by the abnormality detection device 2, and notify the worker 1 himself/herself by the sound of the buzzer 301 which is the alarm part or the vibration of the small motor 304 in the apparatus 3, which enables reliably coping with the abnormality of the body of the worker 1. At least one of the abnormality detection device 2 and the first alarm generation device 3 are owned by the worker himself / herself. It is desirable that the CPU of the abnormality detection device and the CPU of the first alarm generation device 3 are the same, i.e., even in a situation where wireless communication cannot be performed between the first alarm generation device 3 and the second alarm generation device 4, the abnormality of the body of the worker 1 is reliably detected, and the abnormality of the worker 1 can be notified to the worker 1 himself/herself.

In this manner, according to the present invention, abnormality of the body such as heat stroke of the worker 1 is reliably detected, and the abnormality is notified to the worker 1 himself/herself by the sound of the buzzer 301, the light of the light 303, the vibration of the small motor, or the like, which enables reliably responding to the abnormality of the body of the worker 1. That is, even in a situation where the wireless communication cannot be performed between the first alarm generation device 3 of the second alarm generation device 4, the abnormality of the body of the worker 1 can be reliably detected, and the abnormality is notified to the worker 1 himself/herself.

Further, in addition to notifying the worker 1 of the abnormality, the abnormality detection device 2 wirelessly transmits a notification of the abnormality (warning content, warning content) to the remote second alarm generation device 4, so that the administrator 5 can grasp the abnormality from the display of the second alarm generation device 4 and take appropriate measures.

Further, the threshold values for determining the abnormality of respective items are not commonly shared between all the worker, but calculated based on the biological information (steady values) of the worker 1 when the power supply of the abnormality detection device 2 is turned on, which enables reliably detecting the risk of heat stroke at an earlier stage and reduce false alarms.

Further, the abnormality detection device 2 can reduce the consumption amount of the battery 207 by transmitting a radio signal only when it is determined that there is an abnormality in the body of the worker 1, except for periodical transmission of the sensor data. Further, for example, the sensor for acquiring the biological information of the worker 1 may also be a heartbeat sensor, a temperature sensor, a humidity sensor, an acceleration sensor, a perspiration sensor, a blood pressure sensor, etc., biological information, surrounding circumstance information or a combination of two or more of them. Further, these sensors may not necessarily be integrated within the abnormality detection device 2, as long as they are configured to be able to send the predetermined biological information to the abnormality detection device 2.

If it is determined that the body of the worker 1 is abnormal by the abnormality detection device 2, and the worker 1 presses the button 206 within a predetermined time thereafter, the abnormality signal may not be transmitted to the second alarm generation device 4 (i.e., cancellation). However, if the body of the worker 1 obviously has an abnormality, such cancellation is not appropriate, so that cancellation can be made only when the body of the worker 1 is not always abnormal.

In addition, the value of the warning level is not limited to this embodiment, and the administrator 5 can appropriately set the value based on statistical data and the like. Further, the threshold value may be one of a warning level and an alarm level.

Although the embodiment has been described by exemplifying the heat stroke alarm system as an example, such an alarm system can be applied not only to warning of heat stroke but also to warning of various dangers for scenes and people. Also, the protective equipment provided with the warning system is not limited to protective garment, but helmets, gloves, boots, watches, hoofs etc. may be used.

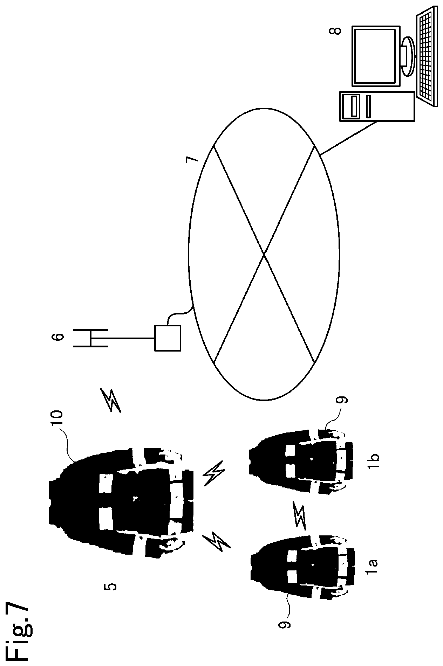

Furthermore, the alarm system according to the present invention can connect to a server or the like located remotely via a network, to accumulate and utilize information. FIG. 7 is a diagram illustrating a system in which the second alarm generation device 4 held by the administrator 5 is connected to a server 8 which is remotely located, via the network 7, such as wireless communications with the base station 6 and Internet. In FIG. 7, biological information, position information, etc. of the workers 1a and 1b are acquired by various sensors provided in the respective abnormality detection devices 2, and are transmitted to the server 8 via the second alarm generation device 4 of the administrator 5; however, in addition to this, a system may be configured such that the first alarm generation devices 3 of the workers 1a and 1b are connected to the server 8 via wireless communication with the base station 6 and the network 7.

For example, the server 8 routinely or periodically measures biological information such as the body temperature, heart rate, blood pressure, respiration rate and the like of the workers 1a and 1b from the sensors of the abnormality detection device 2, calculates an average value in daily life, and determines the thresholds for determining an abnormal for the respective workers 1, which enables adaptation for each worker 1.

Further, if the server 8 routinely or regularly acquires the biological information, the change in the physical condition of the worker 1 is easily noticed. For example, if the alarm system according to the present invention is applied to uniforms such as buses or taxi drivers who take a lot of lives, it is possible to find driver's abnormality at an early stage to take countermeasures.

The normal biological information of the worker 1 accumulated in the server 8 is not limited to being acquired from the sensors of the abnormality detection device 2 but external information such as regular medical examination may be input and used such that the threshold value of the biological information of each worker 1 may be determined based on these items of information.

Further, if the server 8 manages the biological information and the position information of the worker 1, for example, by the server 8 installed in the fire department the position, working environment, etc. of each firefighters in operation can be grasped, and the case where danger is predicted or where there is a firefighter with abnormality in biological information, etc., can be reported from the fire department to the captain of the firefighters who is the administrator 5, which enables reducing the burden on the firefighter commander.

Furthermore, by accumulating the position information (including altitude information) and the biological information data in the server 8, it is possible to investigate and analyze the behavior trajectory of each worker 1 undergoing work and the physical condition at that time. Workers, such as firefighters, SDF, military, rescue team, police officers, security guards, buildings and civil engineering, workers at construction site such as road, etc., can review to improve safety and work efficiency in future activities, or can use as a resource for training and education.

In addition, the biological information, the position information, etc., of the worker 1 may be displayed in the field of view of the own worker or another person's eyeglasses, goggles or the like. Further, each sensor, a battery to be a power source of the sensor, etc., may have a self-diagnosis functional part to have a calibration function such that the calibration function automatically diagnoses and confirms whether it operates normally on the basis of the test signal on a daily basis or just before doing work. If the result of the self-diagnosis is transmitted to the server 8 and accumulated therein, it can be centrally managed as data for the maintenance plan. Further, it is also possible to notify the wearer of the presence or absence of an abnormality by the alarm generation device 3, the alarm generation device 4 or the like, to stop the use.

Specific configurations such as the hardware and flowchart of the alarm system can be appropriately changed without departing from the object of the present invention.

Next, each component will be explained. It is desirable that the temperature sensor for detecting the inner temperature of the protective garment has measurement accuracy of 0.1 °C. As a result, the risk of heat stroke can be detected with high accuracy. As the sensor, a thermocouple or a Peltier element can be used. It is desirable that the temperature sensor is disposed between inter-layers of the multilayer structure fabric or on the skin side surface of the innermost layer. By placing a temperature sensor at a position close to the living body, it is possible to detect the thermal environment that causes the risk of heat stroke. Parts other than the temperature sensor are not necessarily disposed between the inter-layers of the multilayer structure fabric or on the skin side surface of the innermost layer and may be arranged outside the outermost layer. However, in that case, it is desirable that appropriate waterproof treatment is applied to these parts.

The sensor or alarm part or the transmission part or the reception part may be in form of a rectangular parallelepiped, a conical solid, or a flexible form. Examples of flexible forms include plate-like, fibrous, gel-like and the like. By configuring with more flexible materials, it is possible to reduce constraints on workers' activities.

Clothes made of multilayer fabrics are suitably used as articles to which the temperature sensors, the alarm part, the transmission part, or the reception part are applied. Clothes are constantly worn at the time of work so that they are suited for constant monitoring, and are unlikely to obstruct the work and exercise of workers, in contrast with helmets, earphones, etc. However, it goes without saying that this does not exclude installation to a helmet or an earphone, etc., and by installing the sensors, the alarm part, the transmission part, or the reception part at a plurality of points, it is possible to reduce the weight, which leads to an improvement of workability, etc.

In addition, by making the fabric into a multilayer structure, it is possible to simultaneously implementing various functions, which are difficult for a single layer fabric, for a garment. Examples of the functions include flame retardancy, thermal barrier properties, water repellency, chemical permeability, wound resistance, abrasion resistance and the like.

Such multi-layer structure fabric, for example, is preferably one described in Japanese Laid-open Patent Publication No.2014-091307 and Japanese Laid-open Patent Publication No.2011-106069. That is, it is as follows.

The multilayer structure fabric is a laminated fabric comprising two or more layers including an outer layer and an inner layer. In the multilayer structure fabric, it is preferable to use a fiber material having high flame retardancy in order to protect the temperature sensor disposed between the inter-layers or on the skin side surface of the innermost layer of the multilayer structure fabric. For example, the limiting oxygen index (LOI) of the fibers constituting the multilayer structure fabric is 21 or more, preferably 24 or more. The limiting oxygen index is the oxygen concentration (%) of the atmosphere required to continue the combustion, and the limiting oxygen index of 21 or more means that self-extinguishing occurs without continuing combustion in normal air, and thus exhibits increased heat-resistant characteristic. Here, the limiting oxygen index (LOI) is a value measured by JIS L 1091 (Method E).

Thus, by using fibers having an outermost layer having the limit oxygen index (LOI) of 21 or more, high heat resistance can be exhibited. As the above-mentioned fibers, for example, meta-aramid fiber, para-aramid fiber, polybenzimidazole fiber, polyimide fiber, polyamideimide fiber, polyetherimide fiber, polyarylate fiber, polyparaphenylene benzobisoxazole fiber, novoloid fiber, polychlor fibers, flame retardant acrylic fibers, flame retardant rayon fibers, flame retardant polyester fibers, flame retardant cotton fibers, flame retardant wool fibers, and the like are used. In particular, meta-aramid fibers such as polymetaphenylene isophthalamide and para-type aramid fibers for the purpose of improving the strength of the woven or knitted fabric, that is, polyparaphenylene terephthalamide, or fibers obtained by copolymerizing the third component with these fibers are useful. As an example of the polyparaphenylene terephthalamide copolymer, copolyparaphenylene · 3.4'-oxydiphenylene terephthalamide is exemplified. However, easy-to-twist materials such as polyester fiber, polyamide fiber, nylon fiber, and acrylic fiber may be used in combination as long as flame retardancy is not impaired. Further, the fibers may be raw fibers or post-dye fibers. Further, if necessary, a flame-retarding process may be given after weaving the fabric.

For the above-mentioned fibers, long fibers or short fibers may be used. Also, two or more of the above fibers may be mixed or blended and used.

In particular, as the cloth used for the outer layer, in the present invention, the meta-aramid fiber and the para-aramid fiber are preferred to be used in the form of filament or blended spun yarn. The spun yarn used may be single ply or double ply. The mixing ratio of the para type aramid fiber is preferably 5 % by weight or more based on the total fibers constituting the fabric, but since para type aramid fiber is prone to fibrillation, the mixing ratio is 60 % by weight or less.

The cloth may be used in the form of a woven fabric, knitted fabric, nonwoven fabric or the like, but is preferably a woven fabric. As the fabric, any weave structure such as plain weave, twill weave, satin weave or the like may be used. Further, in the case of a woven fabric or a knitted fabric, two kinds of fibers may be used to be combined or interknitted.

It is noted that fabric for use in the outermost layer (Outer layer) has weight per unit area, which is preferably $140 \sim 500 g /m^2$, more preferably $160 \sim 400 g / m^2$, most preferably $200 \sim 400 g / m^2$. When the weight per unit area is less than $140 g / m^2$, sufficient heat resistance may not be obtained. On the other hand, if the weight per unit area exceeds $500 g / m^2$, the feeling of wear in the case of the heat shielding active garment is reduced.

In multilayer constructions fabric, an inner layer is formed by a garment that has tensile modulus of $80 \sim 800 cN$ /dtex, thermal conductivity of $6.0 W \cdot m^{-1} \cdot k^{-1}$ or less, preferably $5.0 W \cdot m^{-1} \cdot k^{-1}$ or less, and a density of $3.0 g / cm^3$,

and electromagnetic wave transmittance at a wavelength of 800 to 3000nm is 10% or less, and weight per unit area is preferably 60 ~ 500 g / m$^2$.

The tensile modulus of elasticity of the fiber is preferably from 80 to 800 cN / dtex (more preferably from 80 to 500 cN / dtex, still more preferably from 120 to 500 cN / dtex). When the tensile elastic modulus is less than 80 cN / dtex, when it is used as a heat shielding activity garment or the like, depending on the wearer's movement and posture, the fiber elongates at a part, the fabric becomes thin, which prevents sufficient heat shielding effect. In addition, when the tensile modulus exceeds 800 cN / dtex, there may be cases where a so-called "stretching" wearing feeling is given. Although it may be possible to avoid this by using a spun yarn, in order to exert a sufficient effect, it is preferable that the tensile elastic modulus is 800 cN / dtex or less.

In the multilayer structure fabric, the weight per unit area of the fabric is preferably 60 to 500 g / m$^2$ (more preferably 80 to 400 g / m$^2$, further preferably 100 to 350 g / m$^2$). If the weight per unit area is less than 60 g / m$^2$, there may be a case where the transmission of electromagnetic waves may not be sufficiently prevented. On the other hand, when the weight per unit area is higher than 500 g / m$^2$, the tendency to accumulate heat becomes conspicuous, there is a possibility that the heat shielding property is hindered, and the light weight property may be impaired.

The fibers constituting the multilayer structure fabric are not particularly limited. In order to improve the absorption and reflection of electromagnetic waves, it is also possible to use metal, carbon or the like which is kneaded or adhered to the surface. Carbon fibers may be used as the above-mentioned fibers, but aramid fibers, polybenzimidazole fibers, polyimide fibers, polyamideimide fibers, polyetherimide fibers, polyarylate fibers, polyparaphenylene benzobisoxazole fibers, novoloid fibers, polychlal fibers, flame-retardant acrylic fibers, flame-retardant rayon fibers, flame-retardant polyester fibers, flame-retardant cotton fibers, fibers comprising an organic polymer such as flame retardant wool fibers (hereinafter sometimes referred to as organic polymer fiber) are suitably used.

In order to improve electromagnetic wave absorptance and thermal conductivity, fine particles of carbon, gold, silver, copper, aluminum and the like may be contained in the organic polymer fiber or adhered to the surface of the organic polymer fiber in the multilayer structure. In this case, carbon or the like may be included in the organic polymeric fiber as a pigment or paint containing the carbon or the like or imparted to the surface of the organic polymeric fiber. The content rate or adhesion rate of these fine particles to the total weight of the organic polymer fiber is preferably from 0.05 to 60 % by weight, more preferably from 0.05 to 40 % by weight, depending on the specific gravity of the fine particles and the like. In the case of carbon particles, it is preferably 0.05 % by weight or more, more preferably 0.05 to 10 % by weight, more preferably more than 0.05 % by weight and less than 5 % by weight. Further, in the case of aluminum fine particles, it is preferably 1 % by weight or more, more preferably 1 to 20 % by weight, further preferably 1 to 10 % by weight.

The number average particle diameter of the fine particles is preferably is 10 μm or less (more preferably 0.01 ~ 1 μ m).

If carbon fiber, metal fiber or the like itself satisfies the above-mentioned requirements such as LOI value, thermal conductivity, etc., it can be used as it is without kneading fine particles. In particular, as a fiber forming the inner layer, a preferable example is a fabric having a content of carbon fibers or metal fibers of preferably 50 % by weight or more, more preferably 80 % by weight or more, further preferably 100 % by weight.

Further, the thickness of each layer of the multilayer structure fabric greatly affects the heat shielding property. For example, as described in Japanese Laid-open Patent Publication No. 2010-255124, it is preferable that the thickness of the outer layer and the thickness of the inner layer satisfy the following formula.

$$5.0 \text{ mm} \geqq \text{ heat shielding layer thickness (mm)} \geqq -29.6 \times (\text{outer layer thickness (mm)}) + 14.1 \text{ (mm)}$$

By using a multilayer structure fabric having high heat shielding property, it is possible to protect the sensor, the alarm part, the transmission part, and the reception part arranged between the inter-layers or on the skin side surface of the innermost layer of the multilayer structure fabric from the flame and reliably transmit alarm information.

In the multilayer structure fabric, the steady state and the fabric form may be changed under the flame exposure. For example, it is considered that the fabric thickness increases steadily under flame exposure. In this way, it is possible to suppress the increase of the risk of heat stroke by being a thin and comfortable cloth structure at steady state, while at the time of flame exposure, the flame protection is enhanced, so that high safety against both heat stroke and flame can be secured.

Also, thermal barrier fabric forming the protective garment preferably has HTI24 of more than 13 seconds as determined by the method specified in IS09151. Thus, in addition to being able to protect the operator from the danger of the flame, it is possible to protect the sensor installed in the clothes, the alarm part, the transmission part, or the reception part from the flame so that they normally exhibit their respective functions.

In addition, the water repellency of the fabric forming the protective garment is preferably higher than the third grade as measured by a spray method prescribed in JIS L 1092. Thus, by protecting the temperature sensor installed in the protective garment from water and liquid chemicals and preventing electric leakage and short circuit, each function can be exhibited normally. A protective garment having high water resistance performance and chemical resistance performance can be obtained by applying a fluorine type water repellent resin to a multilayer structure fabric by a processing method such as a coating method, a spray method, a dipping method or the like. In addition, water repellency may be developed by adding a layer with high water resistance performance to the extent that flame retardancy and heat resistance are satisfied.

In the multilayer structure fabric, the shrinkage ratio is 10% or less (more preferably 5% or less) as the flame resistance, heat resistance and wash resistance heat shrinkage satisfy the international performance standard ISO 11613-1999 applied to the protective garment for firefighters. Furthermore, it is preferable not to ignite, not to separate, not to drip, not to melt, according to the international performance standard ISO 11613-1999. Thereby, it is possible to protect the temperature sensor, the alarm part, the transmission part and the reception part disposed inside the protective garment from the flame and to surely transmit the warning information.

In the multilayer structure fabric, it is also possible to arrange a laminate of a moisture permeable and waterproof film laminated on a fabric made of fibers having an LOI value of 25 or more as an intermediate layer between the outer layer and the inner layer. This makes it possible to suppress the invasion of water from the outside while maintaining the comfort of the fabric structure, so it is more suitable as a protective garment for firefighters who perform firefighting activities such as water discharge. The weight per unit area of the intermediate layer used is preferably in the range of 50 to 200 g / m$^2$. When the weight per unit area is less than 50 g / m$^2$, sufficient heat shielding performance may not be obtained. On the other hand, when the weight per unit area is more than 200 g / m$^2$, the feeling of wear in the case of the heat shielding active garment is inhibited. It is preferable that the cloth is laminated with a thin film made of polytetrafluoroethylene or the like having moisture permeation and waterproof properties, which improves moisture permeation and waterproofness and chemical resistance, and transpiration of the wearer's sweat is promoted, which can reduce the heat stress of the wearer. The weight per unit area of the thin film laminated on the intermediate layer is preferably in the range of 10 to 50 g / m$^2$. Even when a thin film is laminated on the fabric of the intermediate layer in this manner, it is preferable that the weight per unit area of the cloth of the intermediate layer subjected to the processing of the lamination is in the range of 50 to 200 g / m$^2$ described above.

In addition, it is also possible to add a backing layer to the inner side of the inner layer, that is, the skin side, in consideration of practicality such as touch, wearing property and durability in the multilayer structure fabric. The weight per unit area of the fabric used for the backing layer is preferably in the range of 20 to 200 g / m$^2$.

The protective garment can be formed such that, for example, cloth of the outer layer, cloth of the inner layer, cloth of the intermediate layer sandwiched therebetween, if necessary, and cloth which becomes the backing layer further added to the inside of the inner layer as required are superimposed and sewn according to a known method. Further, the laminated fabric of the present invention, the outer layer and the inner layer may be overlapped, fasteners may be attached, these cloths may be sewn, and the fasteners may be removed so that these cloths can be separated as necessary.

By combining the protective garment with the abnormality detection device 2, the first alarm generation device 3, and the second alarm generation device 4, a protective garment having a heat stroke alarm system can be obtained.

Here, it is desirable that the abnormality detection device 2, the first alarm generation device 3, and the second alarm generation device 4 are disposed on the front side of the body of the protective garment. This does not disturb the exercise at the time of activity and prevents personal injury and equipment damage by placing it on the front which is relatively easy to take break -fall, when suddenly overturning or hits the wall.

with respect to the disposing method, various methods can be applied as long as they are attached to protective garment. For example, it is possible to accommodate them in the pocket during sewing, or secure them with a string, a band, a hook and loop fastener, a fastener, a snap button, an adhesive tape, or a bracket. It is also good to stitch them directly or adhere them.

At this time, it is preferable that the abnormality detection device 2 including the sensors is arranged between the inter-layers or on the skin side surface of the innermost layer of the multilayer structure fabric. This is to accurately detect the body temperature rise of the own worker which is a main factor of the heat stroke.

According to the present invention, it is possible to detect not only the danger associated with life such as heat stroke, but also the state of intrusion into the building while ensuring safety, workability, convenience, and it is possible to accurately detect the state of the person who wears the protective equipment. In the present invention, it is also preferable to add an algorithm such that a rescue instruction to the nearest personnel as well as a notification to the emergency services, etc., are implemented.

As described above, the protective garment of the present invention is suitably used as a protective garment for firefighting (fire-fighting garment), but in addition to firefighters, the protective garment of the present invention is suitably used as a protective garment for the SDF, military, rescue team, police officer, security guard, building / civil engineering / road construction, a worker in the field, a worker of a blast furnace, a worker of a nuclear power plant, a worker of a high-temperature environment worker, a farmer, a school activist, a sports competitor, an amusement worker, a care giver requiring watching, an infant, a hospitalized patient, a doctor, a nurse, a caregiver, a pet requiring watching or a livestock, or the like.

[Example]

Next, examples of the present invention will be described in detail, but the present invention is not limited by these examples. Each measurement item in the examples was measured by the method described in Table 1.

(1) Weight per unit area

It was measured according to JIS L 1096-1990.

(2) Thickness

It was measured using a digimatic thickness tester according to JIS L 096-1990 (woven fabric).

(3) Thermal insulation

According to a method based on IS09151, time (HTI24) was measured until the temperature rise reached 24 °C, when it was exposed to the provision of the flame. The longer this time becomes, the better the heat shielding performance becomes.

(4) Thermal Shrinkage Ratio (Dimension Change Rate)

According to ISO 11613, the dimension change rate of the fabric before and after exposure to the heat prescribed in ISO 17493 was measured after prescribed pretreatment.

(5) Heat resistance

According to ISO 11613, it was determined whether it ignited, separated, dropped or melted after being exposed to specified heat.

(6) Water repellency

Water repellency was measured by JIS L1092 (spray method) -1992.

[Example 1]

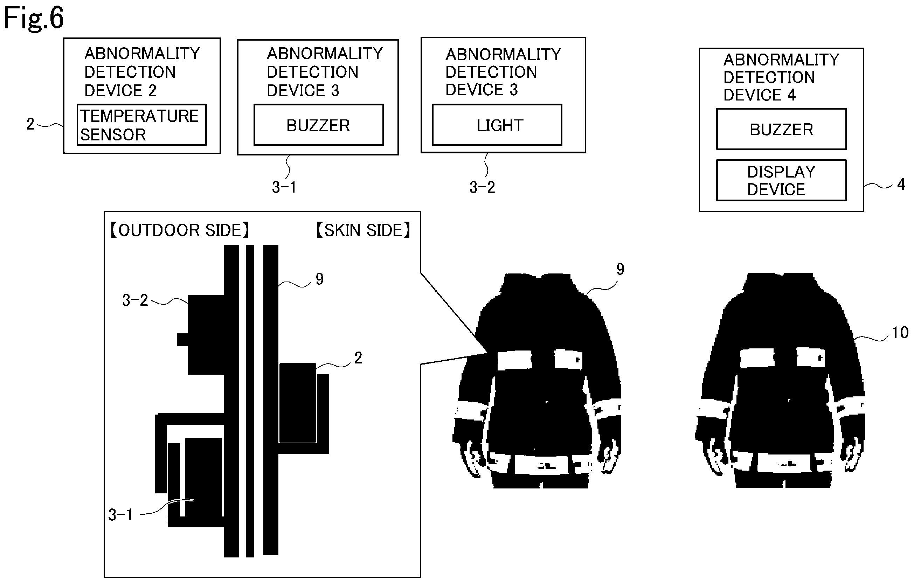

According to the comparative example 4 of Japanese Laid-open Patent Publication No. 2014-091307, a multilayered fabric was obtained and sewn into a shape of a protective garment for fire protection.
Specifically, polymetaphenylene isophthalamide fiber (trade name: Conex, manufactured by Teijin Ltd.) and co-paraphenylene 3, 4'oxydiphenylene terephthalamide fiber (trade name: Technora) were mixed with a mixing ratio of 90:10 to obtain a spun yarn made of heat-resistant fibers (count: 40/2), which was used to weave a fabric having a plain weave ripstop organization. The weight per unit area of the surface layer was 380 g / m$^2$.
For the intermediate layer, polymetaphenylene isophthalamide fiber (trade name: Conex, manufactured by Teijin Ltd.) and co-paraphenylene · 3, 4'oxydiphenylene terephthalamide fiber (Teijin Ltd., trade name: Technora) were mixed with a mixing ratio of 90:10 to obtain a spun yarn made of heat-resistant fibers (count: 40/-) which was woven into a plain weave to be fabric (weight per unit area: 80 g / m$^2$) on which polytetrafluoroethylene moisture-permeable waterproof film (manufactured by Japan Gore-Tex Co., Ltd.) was laminated.
For the heat shielding layer, a filament having a total fineness of 1670 dtex consisting of copolyparaphenylene · 3,4'-oxydiphenylene terephthalamide fiber yarn (trade name: Technora, manufactured by Teijin Ltd.) was used to weave a fabric having a plain weave structure. The weight per unit area of the heat shielding layer (inner layer) was 210 g / m$^2$.
A protective garment was obtained by sewing the multilayer structure fabric. The evaluation results of the protective garment obtained are shown in Table 1.
Next, a unit including the abnormality detection device 2, a unit including a first alarm generation device 3-1 with a buzzer and a display device, and a unit including a first alarm generation device 3-2 with a light were made. A commercial unit including a temperature sensor, a GNSS sensor, a three-axis acceleration sensor, a gyro sensor, a geomagnetic sensor, a pressure sensor, and a transmission part was combined with a determination part and a control part for autonomously estimating the position from the information emitted from the sensors to obtain the abnormality detection device 2. These devices communicated by wireless communication. Subsequently, these were provided in the protective garment for firefighting consisting of multilayered fabrics. The abnormality detection device 2, the first alarm generation device 3-1, and the first alarm generation device 3-2 were placed in the right body central portion of the outerwear of the protective garment for firefighters. Specifically, as shown in FIG. 6, a pocket of the same material as the innermost layer was disposed on the skin side of the innermost layer and the abnormality detection device 2 was accommodated therein. The first alarm generation device 3-1 was disposed on the outer side of the outermost layer of the fire-fighting protective garment, using a pocket. The first alarm generation device 3 - 2 was placed in the waterproof case and then placed on the outer side of the outer layer of the fire-fighting protective garment using a band. This was a protective garment 9 for firefighters used in the example.

Further, a unit including the second alarm generation device 4 was produced. A second alarm generation device 4 was obtained by combining a commercially available unit including reception and transmission parts with a small computer. At this time, a display part indicating that the temperature sensor, the alarm part, the transmission part, and the reception part are operating normally was included. Subsequently, it was placed in the protective garment for firefighters made of multilayer fabric. The second alarm generation device 4 was arranged in the center portion of the right body of the outerwear of the fire protective garment. Specifically, on the skin side of the innermost layer, a pocket of the same material as the innermost layer was arranged and the second alarm generation device 4 was accommodated therein. This was a protective garment 10 for captain of firefighters. The evaluation result is shown in Table 1.

With the protective garment 9 for firefighters and the protective garment 10 for captain of firefighters, it was confirmed that it is possible to detect the position information in the building, and detect the danger of life such as heat stroke to issuer an alert, while securing safety, workability and convenience.

[Example 2]

According to Example 6 of Japanese Laid-open Patent Publication No. 2014-091307, a multilayered fabric was obtained and sewn into the shape of a protective garment for fire protection.

Specifically, polymetaphenylene isophthalamide fiber (trade name: Conex, manufactured by Teijin Ltd.) and co-paraphenylene 3, 4'oxydiphenylene terephthalamide fiber (trade name: Technora) were mixed with a mixing ratio of 90:10 to obtain a spun yarn made of heat-resistant fibers (count: 40/2), which was used to weave a fabric having a plain weave ripstop organization. The weight per unit area of the surface layer was 380 g / m$^2$.

For the intermediate layer, polymetaphenylene isophthalamide fiber (trade name: Conex, manufactured by Teijin Ltd.) and co-paraphenylene · 3, 4'oxydiphenylene terephthalamide fiber (Teijin Ltd., trade name: Technora) were mixed with a mixing ratio of 90:10 to obtain a spun yarn made of heat-resistant fibers (count: 40/-) which was woven into a plain weave to be fabric (weight per unit area: 80 g / m$^2$) on which polytetrafluoroethylene moisture-permeable waterproof film (manufactured by Japan Gore-Tex Co., Ltd.) was laminated.

For the heat shielding layer, aramid fibers containing 1 % by weight of carbon particles in copolyparaphenylene · 3,4'-oxydiphenylene terephthalamide fiber were used. Preparation of polymer solution (dope) and spinning of aramid fibers containing carbon black were carried out by the following method.

2,051 g of N-methyl-2- pyrrolidone (hereinafter referred to as NMP) having a moisture content of about 20 ppm was fed into a mixing tank having an anchor stirring blade flowing nitrogen therein, and 2764 g of paraphenylene diamine and 5114 g of 3,4 '-diaminodiphenyl ether were charged with precisely weighed. The accurately weighed terephthalic acid chloride of 10320 g was fed in the diamine solution in a state of temperature 30 °C and agitation rotational speed of 64 times / min. The temperature of the solution increased to 53 °C due to the heat of reaction and was heated to 85 °C for 60 minutes. Stirring was further continued at 85 °C for 15 minutes to terminate the polymerization reaction upon completion of the viscosity increase of the solution. Thereafter, NMP slurry 16.8kg containing 22.5 % by weight of calcium hydroxide was fed, stirred for 20 minutes to obtain dope of pH5.4, and the dope was filtered by a filter with a mesh size of 30 μm to complete the preparation Polymer solution (hereinafter referred to as dope) of polymer concentration of 6% by weight.

Carbon powder (Dainichiseika Color & Chemicals Mfg. Co., Ltd. "Carbon black FD-0721") was used. The number average particle diameter was 0.36 μm. The carbon particles were added so that the content was 1 % by weight with respect to the fiber.

The addition of carbon black to the fibers is carried out by quantitatively injecting NMP slurry of carbon black into the above-mentioned dope being fed to the carbon black blended spinning head, immediately subjecting to dynamic mixing, followed by a static mixer of 20 or more stages, then discharging from a pack / spinning nozzle via a metering pump, take out by dry jet spinning, winding up the product through coagulation, drying, hot drawing, finishing oil application and winding up the product, to obtain a mixture of copolyparaphenylene · 3, 4'- oxy diphenylene terephthalamide fiber yarn. Using this filament having a total fineness of 1670 dtex, a fabric having a plain weave structure was woven. The weight per unit area of the inner layer was 210 g / m$^2$. The evaluation results are shown in Table 1.

A protective garment was obtained by sewing the multilayer structure fabric. The evaluation results of the protective garment obtained are shown in Table 1.

Next, a unit including the abnormality detection device 2, a unit including a first alarm generation device 3-1 with a buzzer and a display device, and a unit including a first alarm generation device 3-2 with a light were made. A commercial unit including a temperature sensor, a GNSS sensor, a three-axis acceleration sensor, a gyro sensor, a geomagnetic sensor, a pressure sensor, and a transmission part was combined with a determination part and a control part for autonomously estimating the position from the information emitted from the sensors to obtain the abnormality detection device 2. These devices communicated by wireless communication.

Subsequently, it was placed in the fire-fighting protective garment made of multilayer fabric. The abnormality detection device 2, the first alarm generation device 3-1, and the first alarm generation device 3-2 were placed in the right body central portion of the outerwear of the fire-fighting protective garment. Specifically, on the skin side of the innermost layer, a pocket of the same material as the innermost layer was arranged and the abnormality detection device 2 was accommodated therein. The first alarm generation device 3-1 was disposed on the outer side of the outermost layer of the fire-fighting protective garment, using a pocket. The first alarm generation device 3-1 was disposed on the outer side of the outermost layer of the fire-fighting protective garment, using a pocket. This was a protective garment 9 for firefighters used in the example.

Further, a unit including the second alarm generation device 4 was produced. A second alarm generation device 4 was obtained by combining a commercially available unit including reception and transmission parts with a small computer. At this time, a display part indicating that the temperature sensor, the alarm part, the transmission part, and the reception part are operating normally was included. Subsequently, this was provided in the protective garment for firefighting consisting of multilayered fabrics. The second alarm generation device 4 was arranged in the center portion of the right body of the outerwear of the fire protective garment. Specifically, a snap button was attached on the skin side of the innermost layer, and the second alarm generation device 4 which also have a snap button therein was combined by the snap buttons. This was a protective garment 10 for captain of firefighters.

With the protective garment 9 for firefighters and the protective garment 10 for captain of firefighters, it was confirmed that it is possible to detect the position information in the building, and detect the danger of heat stroke to issuer an alert, while securing safety, workability and convenience.


[Example 3]


According to comparative example 1 of Japanese Laid-open Patent Publication No. 2014-091307, a multilayered fabric was obtained and sewn into the shape of a protective garment for fire protection.

Specifically, a dual structure fabric is used as the outermost layer, a spun yarn made of heat-resistant fibers (count: 40/2), which has polymetaphenylene isophthalamide fiber (trade name: Conex, manufactured by Teijin Ltd.) and co-paraphenylene · 3, 4'oxydiphenylene terephthalamide fiber (trade name: Technora) mixed with a mixing ratio of 90:10, was provided on the outer side, and a plain weave using a spun yarn (count : 40 / -) of 100% co-paraphenylene · 3, 4 'oxydiphenylene terephthalamide fiber (Teijin Ltd., trade name: Technora) was provided on the inner side, to be weaved. The outer and inner fabrics were bonded in a lattice structure by the inner Technora (trademark) such that the lattice spacing was 20 mm. The weight per unit area of the surface layer was 205 g / m$^2$.

For the intermediate layer, polymetaphenylene isophthalamide fiber (trade name: Conex, manufactured by Teijin Ltd.) and co-paraphenylene · 3, 4 'oxydiphenylene terephthalamide fiber (Teijin Ltd., trade name: Technora) were mixed with a mixing ratio of 90:10 to obtain a spun yarn made of heat-resistant fibers (count: 40/-) which was woven into a plain weave to be fabric (weight per unit area: 80 g / m$^2$) on which polytetrafluoroethylene moisture-permeable waterproof film (manufactured by Japan Gore-Tex Co., Ltd.) was laminated.

For the heat shielding layer, a spun yarn 1 made of heat-resistant fibers (count: 40/-) 1, which has polymetaphenylene isophthalamide fiber (trade name: Conex, manufactured by Teijin Ltd.) and co-paraphenylene · 3, 4 'oxydiphenylene terephthalamide fiber (Teijin Ltd., trade name: Technora) mixed with a mixing ratio of 95:5, and a spun yarn 2 including a 56 dtex / 12 filament of polyethylene terephthalate fibers (YHY N800SSDC; manufactured by Teijin Co.) and the spun yarn 1 such that they are twisted 500 times in the S-direction were woven with weave density of 113 per 2.54 cm in a latitude direction and 80 per 2.54 cm in a longitude direction to obtain a fabric, and then the fabric was subject to desizing for a minute at 180 °C or desizing was carried out for the final set at 180 °C for 1 minute. A protective garment was obtained by sewing the multilayer structure fabric. The evaluation results are shown in Table 1.

Next, an abnormality detection device 2, a first alarm generation device 3-1 with a buzzer and a display device, and a first alarm generation device 3-2 with a light were made. A commercial unit including a temperature sensor, a GNSS sensor, a three-axis acceleration sensor, a gyro sensor, a geomagnetic sensor, and a transmission part was combined with a determination part and a control part for autonomously estimating the position from the information emitted from the sensors to obtain the abnormality detection device 2. These devices communicated by wireless communication.

Subsequently, it was placed in the protective garment for firefighters made of multilayer fabric. The abnormality detection device 2, the first alarm generation device 3-1, and the first alarm generation device 3-2 were placed in the right body central portion of the outerwear of the protective garment for firefighters. Specifically, on the skin side of the innermost layer, a pocket of the same material as the innermost layer was arranged and the abnormality detection device 2 was accommodated therein. The first alarm generation device 3-1 was disposed on the outer side of the outermost layer of the fire-fighting protective garment, using a pocket. The first alarm generation device 3 - 2 was placed in the waterproof case and then placed on the outer side of the outer layer of the fire-fighting protective garment using a band. This was a protective garment 9 for firefighters used in the example.

Further, the second alarm generation device 4 was produced. A second alarm generation device 4 was obtained by combining a commercially available unit including reception and transmission parts with a small computer. At this time, a display part indicating that the temperature sensor, the alarm part, the transmission part, and the reception part are operating normally was included. Subsequently, it was placed in the protective garment for firefighters made of multilayer fabric. The second alarm generation device 4 was arranged in the center portion of the right body of the outerwear of the fire protective garment. Specifically, a surface fastener was attached on the skin side of the innermost layer, and the second alarm generation device 4 which also have a surface fastener therein was combined by the surface fasteners. This was a protective garment 10 for captain of firefighters.

With the protective garment 9 for firefighters and the protective garment 10 for captain of firefighters, it was confirmed that it is possible to detect the position information in the building, and detect the danger of heat stroke to issuer an alert, while securing safety, workability and convenience.

[Example 4]

According to the comparative example 4 of Japanese Laid-open Patent Publication No. 2014-091307, a multilayered fabric was obtained and sewn into a shape of a protective garment for fire protection.

Specifically, polymetaphenylene isophthalamide fiber (trade name: Conex, manufactured by Teijin Ltd.) and co-paraphenylene 3, 4 'oxydiphenylene terephthalamide fiber (trade name: Technora) were mixed with a mixing ratio of 90:10 to obtain a spun yarn made of heat-resistant fibers (count: 40/2), which was used to weave a fabric having a plain weave ripstop. The weight per unit area of the surface layer was 380 g / m$^2$.

For the heat shielding layer, a filament having a total fineness of 1670 dtex consisting of copolyparaphenylene · 3,4'-oxydiphenylene terephthalamide fiber yarn (trade name: Technora, manufactured by Teijin Ltd.) was used to weave a fabric having a plain weave. The weight per unit area of the heat shielding layer (inner layer) was 210 g / m$^2$. The evaluation results are shown in Table 1.

A protective garment was obtained by sewing the multilayer structure fabric. Aside from this, as in the case of Example 1, it was found that the waterproofness of the multilayered fabric was not sufficient, the temperature sensor, the GNSS sensor, the three axis acceleration sensor, the gyro sensor, the geomagnetic sensor, the alarm part, transmission part, and the reception part installed inside were wet when water was discharged, in the situation where there was no water wetness, the system detected the position information in the building, and it was able to detect the danger of heat stroke and alarm it.

[Example 5]

In Example 1, the same as Example 1 except that the GNSS sensor was changed to one including part configured to measure position uncertainty.

[Example 6]

A protective garment was obtained in the same manner as in Example 1.

Next, a unit including the abnormality detection device 2, a unit including a first alarm generation device 3-1 with a buzzer and a display device, and a unit including a first alarm generation device 3-2 with a light were made. A commercial unit including a temperature sensor, a GNSS sensor, a three-axis acceleration sensor, a gyro sensor, a geomagnetic sensor, a pressure sensor, and a transmission part was combined with a determination part and a control part for autonomously estimating the position from the information emitted from the sensors to obtain the abnormality detection device 2. These devices communicated by wireless communication.

Meanwhile, a radio wave transmission/indoor position information grasping system was obtained by combining an emergency exit light set in a building with a beacon.

Subsequently, these were provided in the protective garment for firefighting consisting of multilayered fabrics. The abnormality detection device 2, the first alarm generation device 3-1, and the first alarm generation device 3-2 were placed in the right body central portion of the outerwear of the fire-fighting protective garment. Specifically, on the skin side of the innermost layer, a pocket of the same material as the innermost layer was arranged and the abnormality detection device 2 was accommodated therein. The first alarm generation device 3-1 was disposed on the outer side of the outermost layer of the fire-fighting protective garment, using a pocket. The first alarm generation device 3-1 was disposed on the outer side of the outermost layer of the fire-fighting protective garment, using a band. This was a protective garment 9 for firefighters used in the example.

Further, a unit including the second alarm generation device 4 was produced. A second alarm generation device 4 was obtained by combining a commercially available unit including reception and transmission parts with a small computer. At this time, a display part indicating that the temperature sensor, the alarm part, the transmission part, and the reception part are operating normally was included. Subsequently, it was placed in a protective garment for firefighters made of multilayer fabric. The second alarm generation device 4 was arranged in the center portion of the right body of the outerwear of the fire protective garment. Specifically, on the skin side of the innermost layer, a pocket of the same material as the innermost layer was arranged and the second alarm generation device 4 was accommodated therein. This was a protective garment 10 for captain of firefighters. With the protective garment 9 for firefighters and the protective garment 10 for captain of firefighters, it was confirmed that it is possible to detect the position information in the building, and detect the danger of life such as heat stroke to issuer an alert, while securing safety, workability and convenience.

[Table 1]

| | OUTER LAYER | | | | INTERMEDIATE LAYER | | | | INNER LAYER | | | | PROTECTIVE GARMENT FOR FIREFIGHTING | | |
|---|---|---|---|---|---|---|---|---|---|---|---|---|---|---|---|
| | WEIGHT PER UNIT AREA (g/cm2) | WOVEN STRUCTURE | SHRINKAGE RATIO (%) | HEAT RESISTANCE | WEIGHT PER UNIT AREA (g/cm2) | SHRINKAGE RATIO (%) | HEAT RESISTANCE | MATRIX MATERIAL | WEIGHT PER UNIT AREA | WOVEN STRUCTURE | SHRINKAGE RATIO (%) | HEAT RESISTANCE | TOTAL WEIGHT PER UNIT AREA (g·cm2) | HEAT SHIELDING PERFORMANCE (HT124) | WATER REPELLENCY |
| EXAMPLE 1 | 380 | PLAIN WEAVE RIP | <5% | CLEARED | 120 | <5% | CLEARED | ARAMID | 210 | PLAIN WEAVE | <5% | CLEARED | 590 | 15.8 | FOURTH GRADE |
| EXAMPLE 2 | 380 | PLAIN WEAVE RIP | <5% | CLEARED | 120 | <5% | CLEARED | ARAMID/CARBON (1%) | 210 | PLAIN WEAVE | <5% | CLEARED | 590 | 16.5 | FOURTH GRADE |
| EXAMPLE 3 | 205 | DOUBLE WEAVE | <5% | CLEARED | 120 | <5% | CLEARED | ARAMID | 290 | DOUBLE WEAVE | <5% | CLEARED | 451 | 20.1 | FOURTH GRADE |
| EXAMPLE 4 | 380 | PLAIN WEAVE RIP | <5% | CLEARED | - | - | - | | 210 | PLAIN WEAVE | <5% | CLEARED | 590 | 15.8 | SECOND-THIRD GRADE |

Industrial Applicability

INDUSTRIAL APPLICABILITY According to the present invention, it is possible to provide an alarm system capable of detecting positional information in a building, detecting a danger of life such as heat stroke, and issuing an alarm while detecting safety, workability and convenience, and its industrial value is extremely high.

Description of Reference Symbols

2    Abnormality detection device
3    first alarm generation device
4    second alarm generation device
7    Network
8    servers
9    Protective garment for firefighters
10    Protective garment for captain of firefighters

**Claims**

1.  An article including an alarm system, **characterized in that** the alarm system includes a position information sensor.

2.  The article of claim 1, wherein the alarm system includes, in addition to the position information sensor,
    a three-axes acceleration sensor, a gyro sensor, a geomagnetic sensor, or a combination thereof; and
    a part configured to autonomously estimate a position from information emitted from the position information sensor.

3.  The article comprising an alarm system according to claim 2, wherein the alarm system further includes an atmospheric pressure sensor.

4.  The article comprising an alarm system according to claim 2, wherein the alarm system further includes;
    a biometric information sensor that detects biometric information of a wearer of the article;
    a determination part configured to determine that the biological information detected by the biometric information sensor has reached a threshold value;
    an alarm part configured to warn that a risk is heightened in response to an instruction from the determination part;
    a transmission part configured to transmit a warning in response to the alarm part being operating; and
    a control part configured to control the alarm part and the transmission part.

5.  The article according to claim 4, wherein the biometric information sensor is a temperature sensor that detects an inside temperature of the article.

6.  An article including an alarm system, **characterized in that** the alarm system includes;
    a reception part configured to receive the alarm transmitted from the transmission part according to claim 4;
    an alarm part;
    a display part; and
    a control part configured to control the reception part and the alarm part.

7.  The article according to claim 4 or 6, wherein the alarm part is based on a voice, light, a display on a display part, a vibration of the sensor itself, or a combination thereof.

8.  The article according to claim 4 or 6, comprising a display part configured to indicate that the sensor, the determination part, the alarm part, the transmission part, or the reception part is operating normally.

9.  The article according to claim 1, wherein the alarm system includes;
    a position information sensor,
    a calculation part configured to calculate a position uncertainty degree from information generated from the position information sensor;
    an alarm part configured to alarm the position uncertainty degree calculated by the calculation part;

a transmission part configured to transmit the alarm upon the alarm part is activated; and
a control part configured to control the alarm part and the transmission part.

10. The article according to claim 1, wherein the alarm system includes,
a position information sensor,
a three-axes acceleration sensor, a gyro sensor, a geomagnetic sensor, or a combination thereof;
a part configured to autonomously estimate a position from information emitted from the position information sensor; and
a part configured to increase position information accuracy within a building by using a position information detection system provided in the building.

11. The article according to claim 1, wherein the article is a garment constituted by a multilayer-structured cloth.

12. The article according to claim 11, wherein the sensor is provided on an interlayer of the multilayer-structured cloth or a surface of an innermost layer on a skin side.

13. The article according to claim 11, wherein the cloth constituting the garment has such a heat shielding property that HTI 24 is 13 seconds or more as measured by a method prescribed in ISO 9151.

14. The article according to claim 11, wherein an outermost surface of the cloth constituting the garment has a water repellency of class 3 or higher as measured by a spray method prescribed in JIS L1092.

15. The article according to claim 11, wherein the cloth constituting the garment has a heat shrinkage percentage of 10% or less according to an international performance standard ISO 11613-1999.

16. The article according to claim 11, wherein the garment is a fire protective garment.

17. The article according to claim 11, wherein the garment is used for one selected from a group, the group including a member of SDF (Self - Defense Forces), military, a rescue team, police officers, security guards, construction workers, civil engineering workers, road construction site workers, workers at blast furnaces, workers at nuclear power plants, workers at high temperatures, agricultural workers, school activists, sports athletes, amusement workers, care receivers requiring watching, infants, inpatients, doctors, nurses, caregivers, pets or livestock requiring watching.

18. The article according to claim 11, wherein the cloth constituting the garment includes aramid fibers.

Fig.1

# Fig.2

201 : TEMPERATURE SENSOR
202 : CPU (PROCESSING PART)
203 : WIRELESS MODULE
204 : MEMORY (STORAGE PART)
205 : RTC
206 : BUTTON
207 : BATTERY

301 : BUZZER
302 : CPU
303 : LIGHT
304 : SMALL MOTOR
305 : WIRELESS MODULE
306 : BATTERY

401 : COMPUTER
402 : BUZZER
403 : WIRELESS MODULE
404 : MEMORY DEVICE
405 : INPUT DEVICE
406 : BATTERY
411 : PROCESSING UNIT
412 : STORAGE UNIT
413 : DISPLAY UNIT

# Fig.3

```
┌─────────────────────────┐
│  ABNORMALITY DETECTION  │
│  DEVICE 2               │
└─────────────────────────┘

┌─────────────────────────┐  S1
│  POWER SUPPLY TURNED ON  │
└─────────────────────────┘
              │
              ▼
┌─────────────────────────┐  S2
│ SENSOR DATA MEASURED AND │
│ STORED IN NORMAL STATE   │
└─────────────────────────┘
              │
              ▼            S3
        ╱───────────╲
       ╱ MEASUREMENT ╲◄──────────┐
       ╲   TIMING?   ╱           │
        ╲───────────╱            │
           │ Yes                 │
   S4      ▼                     │
┌─────────────────┐              │
│ SENSOR DATA     │              │
│ COLLECTED AND   │              │
│ ACCUMULATED     │              │
└─────────────────┘              │
   S5      │                     │
           ▼            No       │
        ╱───────────╲ ──────────┘
       ╱ ALERT LEVEL ╲
       ╲  EXCEEDED?  ╱
        ╲───────────╱
           │ Yes
   S6      ▼
┌─────────────────┐
│ ABNORMALITY     │
│ CONTENT         │───────┐
│ TRANSMITTED     │       │
└─────────────────┘       │
```

```
┌──────────────────────────┐    ┌──────────────────────────┐
│ FIRST ALARM GENERATION   │    │ SECOND ALARM GENERATION  │
│ DEVICE 3                 │    │ DEVICE 4                 │
└──────────────────────────┘    └──────────────────────────┘
            │                               │
            ▼                               ▼
      ╱───────────╲                   ╱───────────╲
     ╱   ALERT     ╲                 ╱   ALERT     ╲
     ╲  CONTENT    ╱                 ╲  CONTENT    ╱
      ╲ RECEIVED  ╱                   ╲ RECEIVED  ╱
       ╲─────────╱                     ╲─────────╱
          │ Yes                           │ Yes
          ▼                               ▼
┌──────────────────┐            ┌──────────────────┐
│ ALARM APPARATUS  │            │ ALARM APPARATUS  │
│ OPERATED         │            │ OPERATED         │
└──────────────────┘            └──────────────────┘
```

EP 3 447 745 A1

Fig.4

| WORKER | DETERMINATION CYCLE (SECOND) | TIME | TEMPERATURE IN GARMENT (°C) | RECORD VALUE ALARM LEVEL | DETERMINATION |
|---|---|---|---|---|---|
| 1a | 10 | 13:50:30 | 32 | | NORMAL |
| | | | 38 | | |
| 1a | 10 | 13:50:40 | 32 | | NORMAL |
| | | | 38 | | |
| | | | | | |
| | | | | | |

EP 3 447 745 A1

Fig.5

EP 3 447 745 A1

| WORKER | TIME | TEMPERATURE IN GARMENT (°C) | NOMINAL VALUE | DETERMINATION |
|---|---|---|---|---|
| | | | ALARM LEVEL | |
| 1a | 13:50:30 | 32 | | NORMAL |
| | | 38 | | |

# Fig.6

| ABNORMALITY DETECTION DEVICE 2 | ABNORMALITY DETECTION DEVICE 3 | ABNORMALITY DETECTION DEVICE 3 | ABNORMALITY DETECTION DEVICE 4 |
|---|---|---|---|
| TEMPERATURE SENSOR | BUZZER | LIGHT | BUZZER |
| | | | DISPLAY DEVICE |

2

3-1

3-2

4

【OUTDOOR SIDE】　　　【SKIN SIDE】

3-2

9

2

3-1

9

10

EP 3 447 745 A1

EP 3 447 745 A1

Fig.7

## INTERNATIONAL SEARCH REPORT

| | International application No. |
|---|---|
| | PCT/JP2017/014617 |

| A. CLASSIFICATION OF SUBJECT MATTER |
|---|
| *G08B21/02*(2006.01)i, *A41D13/00*(2006.01)i, *A61B5/00*(2006.01)i, *B32B5/26* (2006.01)i, *D03D1/00*(2006.01)i, *D03D11/00*(2006.01)i, *D03D15/00*(2006.01)i, *G08B19/00*(2006.01)i, *G08B25/04*(2006.01)i, *G08B25/10*(2006.01)i |
| According to International Patent Classification (IPC) or to both national classification and IPC |

| B. FIELDS SEARCHED |
|---|
| Minimum documentation searched (classification system followed by classification symbols) |
| G08B21/02, A41D13/00, A61B5/00, B32B5/26, D03D1/00, D03D11/00, D03D15/00, G08B19/00, G08B25/04, G08B25/10 |

| Documentation searched other than minimum documentation to the extent that such documents are included in the fields searched |
|---|
| Jitsuyo Shinan Koho        1922–1996   Jitsuyo Shinan Toroku Koho   1996–2017 |
| Kokai Jitsuyo Shinan Koho   1971–2017   Toroku Jitsuyo Shinan Koho    1994–2017 |

| Electronic data base consulted during the international search (name of data base and, where practicable, search terms used) |
|---|
| |

### C. DOCUMENTS CONSIDERED TO BE RELEVANT

| Category* | Citation of document, with indication, where appropriate, of the relevant passages | Relevant to claim No. |
|---|---|---|
| X | JP 2005-146499 A  (Hideo HIROSE), 09 June 2005 (09.06.2005), | 1,11,12,17, 18 |
| Y | paragraphs [0046] to [0059], [0064], [0074], [0106]; fig. 10 (Family: none) | 2-10,13-16 |
| Y | JP 2014-96781 A  (Ricoh Co., Ltd.), 22 May 2014 (22.05.2014), paragraphs [0196], [0197] & US 2014/0106777 A1 paragraphs [0247], [0248] | 2-8,10 |
| Y | JP 2005-128706 A  (Kabushiki Kaisha Kokusai Joho Research Kenkyusho), 19 May 2005 (19.05.2005), paragraphs [0016] to [0025]; fig. 1, 2 (Family: none) | 6-8 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 June 2017 (14.06.17) | 27 June 2017 (27.06.17) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2017/014617 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2006-62567 A  (Kojima Press Industry Co., Ltd.),<br>09 March 2006 (09.03.2006),<br>paragraph [0013]<br>(Family: none) | 8 |
| Y | JP 2005-77211 A  (Matsushita Electric Industrial Co., Ltd.),<br>24 March 2005 (24.03.2005),<br>paragraph [0027]<br>(Family: none) | 9 |
| Y | JP 2004-30180 A  (Masami MURAYAMA),<br>29 January 2004 (29.01.2004),<br>paragraphs [0013] to [0024], [0028]; fig. 1 to 3<br>(Family: none) | 13-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013048812 A
- JP 2012187127 A
- JP 2004030180 A
- JP 2014091307 A
- JP 2011106069 A
- JP 2010255124 A